# EUROPÄISCHE PATENTANMELDUNG

(11) **EP 1 859 738 A1**
(43) Veröffentlichungstag der Anmeldung: **28.11.2007**
(21) Anmeldenummer: 06010903.0
(22) Anmeldetag: 27.05.2006
(51) Int. Cl.: A61B 5/11, A61B 5/00

(54) **Tragbares diagnostisches System für Gleichgewichtsfunktion**

(71) Anmelder: Ernst, Arneborg, 14169 Berlin (DE); Basta, Dietmar, 14656 Brieselang (DE)
(72) Erfinder: Ernst, Arneborg, 14169 Berlin (DE); Basta, Dietmar, 14656 Brieselang (DE)
(74) Vertreter: Boeckh, Tobias

(57) **Zusammenfassung**

Die Erfindung betrifft eine mobile Gleichgewichtsprothese sowie die Verwendung der Gleichgewichtsprothese zum Gleichgewichtstraining eines Körpers; weiterhin betrifft die Erfindung ein Verfahren zum mobilen Gleichgewichtstraining und die Erfindung betrifft auch die Verwendung eines Gyrometers bei mobilen Gleichgewichtsprothesen.

## Beschreibung

Die Erfindung betrifft eine mobile Gleichgewichtsprothese sowie die Verwendung der Gleichgewichtsprothese zum Gleichgewichtstraining eines Körpers; weiterhin betrifft die Erfindung ein Verfahren zum mobilen Gleichgewichtstraining und die Erfindung betrifft auch die Verwendung eines Gyrometers bei mobilen Gleichgewichtsprothesen.

Alle Organismen, insbesondere die Wirbeltiere und hier wiederum insbesondere die aufrecht gehenden, verfügen über einen speziellen Gleichgewichtssinn. Der Gleichgewichtssinn dient zur Feststellung der Körperhaltung und Orientierung im Raum. Er hat beispielsweise beim Menschen sein Zentrum im Gleichgewichtsorgan, im Innenohr und im Kleinhirn, wobei er aber eng mit den Augen und anderen Sinnen sowie Reflexen verbunden ist. Zum Gleichgewichtssinn gehört das Empfinden für oben und unten (Lotrichtung), für Winkel bzw. Neigungen (Lageorientierung) und Rhythmus sowie Linear- und Drehbeschleunigungen insbesondere des Kopfes in alle Richtungen. Der Gleichgewichtssinn des Menschen wird komplettiert durch den Gesichtssinn (zur Raumlage), durch die Muskulatur des Skeletts (bei Körperdehnungen und Beschleunigungen), das Gesäß (bei Beschleunigung vor allem in vertikaler Richtung), das Gehör (zur Einschätzung von Geschwindigkeit mithilfe von Luftgeräuschen) sowie den Hautsinn (für Eigen- und Luftbewegungen bei Linear- und Drehbeschleunigung).

Stoffwechseländerungen, Erkrankungen, Unfälle aber auch natürliche Vorgänge wie das Altern können den Gleichgewichtssinn stören.

Im Stand der Technik sind mehrere Möglichkeiten beschrieben, einen gestörten Gleichgewichtssinn zu therapieren bzw. den Patienten so zu trainieren, dass er durch den gestörten Gleichgewichtssinn nur bedingt eingeschränkt ist. Wenn beispielsweise die Augenbewegung und Haltungsreaktion außerhalb der Grenzwerte für eine gute Gleichgewichtsorientierung liegen, kann mithilfe von Medikamenten die Tätigkeit der peripheren Gleichgewichtssinne im Gehirn reduziert werden. Die betroffene Person kann dann das eigene Gehirn darauf trainieren, mit einem verminderten Gleichgewichtssinn den Körper aufrecht zu halten. Die Medikamente weisen jedoch zahlreiche Nebenwirkungen auf, so dass diese Methode nicht immer angewandt werden kann.

Im Stand der Technik sind zahlreiche Vorrichtungen beschrieben, mit denen der Gleichgewichtssinn trainiert werden kann. So beschreibt die US 4092633 ein System zur Messung des Bewegungsstatus mit einem Detektor, der einen Puls erzeugt, wenn ein Parameter eines überwachten Bewegungsstatus eine definierte Schwelle überschreitet. Ein rücksetzbarer Bewegungsstatusmesser zählt jeden Puls, wobei ein gewisser Zeitrahmen durch einen Zeitmesser vorgegeben wird. Der Bewegungsstatusmesser erzeugt ein Signal, wenn die Gesamtzahl der Pulse innerhalb des Zeitrahmens größer oder gleich der vorgegebenen Anzahl ist. Eine Überwachungsvorrichtung erzeugt als Antwort auf die einlaufenden Pulse und das Bewegungsstatussignal Steuersignale, welche die Vorrichtung kontrollieren.

Weitere Vorrichtungen zur Auswertung von charakteristischen Bewegungen, die mit dem Gleichgewichtssinn assoziiert sind, sind beispielsweise in der US 5361778, US 5469861 und in der WO 8804909 beschrieben.

Die DE 3416837 offenbart ein System zur Erlangung einer aufrechten Gehhaltung, bei dem Sensoren und Nervenstimulatoren verwendet werden, wobei die Vorrichtung mittels eines Computers gesteuert wird. Die US 5281957 offenbart einen tragbaren Computer und eine am Kopf montierte, durchsichtige Anzeige in Form einer Brille, bei der die Linsen in Form einer Flüssigkeitskristallanzeige ausgebildet sind.

Es ist bekannt, dass die Funktion des menschlichen Gleichgewichtsorgans objektiv durch die Kontrolle verschiedener vestibulärer Reflexe sowie durch die Testung der Haltungsreaktionen in bestimmten Bewegungs- bzw. Testsituationen quantifizierbar ist. Dabei wird geprüft, ob die bei normaler Gleichgewichtsfunktion zu erwartenden Normbereiche eingehalten werden. Bei signifikanten Abweichungen von diesen Normbereichen wird, entsprechend der diagnostizierten spezifischen Fehlfunktion, meistens eine medikamentöse, chirurgische oder physiotherapeutische Behandlung eingeleitet. Diese Methoden sind derzeit jedoch nicht in jedem Fall erfolgreich. So ist eine medikamentöse Behandlung (siehe oben) nicht immer spezifisch möglich und bewirkt daher zumeist nur eine Abschwächung der Schwindelbeschwerden.

Chirurgische Eingriffe stellen aufgrund möglicher perioperativer Komplikationen nur bei sehr wenigen Erkrankungen eine Methode der Wahl dar. Im Rahmen der Behandlung wird meist zusätzlich ein physiotherapeutisch ausgerichtetes Gleichgewichtstraining absolviert. Während solcher Trainingseinheiten setzt sich der Patient wiederholt definierten, das Gleichgewichtssystem destabilisierenden Grenzsituationen aus mit dem Ziel, die Gleichgewichtskontrolle zu verbessern. Im Laufe dieser Übungen (mehrere Monate) kommt es beim Patienten zu einer mehr oder weniger ausgeprägten zentralnervösen Kompensation der veränderten Gleichgewichtsinformation unter Zuhilfenahme anderer sensorischer Modalitäten (optisch, somatosensorisch). Bei vielen Patienten reicht, aufgrund von unklaren Therapieresistenzen oder der Schwere der Erkrankung, ein solches Training zur vestibulären Rehabilitation allerdings nicht aus. Dennoch konnte eine Verbesserung der Gleichgewichtskontrolle bei diesen Patienten mit Hilfe von Geräten erreicht werden, die Informationen über die Positionsänderung des Körpers in einen kompensatorischen Stimulus (z.B. optisch, akustisch) umkodieren und diesen dann intakten sensorischen Eingängen des Patienten (z.B. Auge, Ohr) verstärkt darbieten. Bisher ist die Therapie ortsgebunden und nur für ein Training mit bestimmten vorgegebenen Aufgaben geeignet. Ein alltagstaugliches, mobiles Gerät zur individuellen Unterstützung des Gleichgewichtssystems in allen Alltagssituationen (z. B. auch beim Jogging, Fahrradfahren usw.) ist derzeit nicht verfügbar. Ein solches Gerät sollte vor allem in der Lage sein, willkürliche Bewegungen des Trägers von Bewegungen zu unterscheiden, die durch Gleichgewichtsstörungen verursacht werden. Bisherige Geräte sind dazu nicht in der Lage. Zudem dürfen die verbliebenen Sinneseindrücke nicht durch die Verwendung des Gerätes behindert bzw. beeinflusst werden. Diese Anforderungen werden bisher auch von keinem Gerät erfüllt. Aktuelle Geräte haben nur eine eingeschränkte Trainings- oder Prothesefunktion während einer bestimmten Aufgabe. Außerdem verhindert bei diesen Geräten die Art der Stimulation (optisch, gustatorisch oder akustisch) zusätzlich den Alltagsgebrauch. In bisherigen Geräten wird häufig u. a. die Beschleunigung von Körperteilen gemessen und beim Überschreiten von Schwellenwerten eine Signal bereitgestellt. Beim Laufen, Fahrradfahren oder ähnlichen Aktivitäten treten lineare Beschleunigungen auf, die nichts mit der Aufrechterhaltung des Gleichgewichtes zu tun haben. Hat diese Beschleunigung Einfluss auf den angebotenen kompensatorischen Stimulus, ist das Gerät nicht geeignet, in Alltagssituationen eingesetzt zu werden.

Die im Stand der Technik bekannten pharmazeutischen Mittel, wie auch Vorrichtungen und Verfahren, erlauben es aber nicht, fehlende Gleichgewichts- oder Raumorientierungsinformationen in alltäglichen Situationen zu kompensieren, mit deren Hilfe das Gehirn die Körperhaltung im Raum bestimmen könnte und so eine notwendige Haltungskorrektur einleiten kann. Die im Stand der Technik bekannten Vorrichtungen erlauben es auch nicht, sie als Trainingsgerät im Rahmen der Rehabilitation bei Gleichgewichtsstörungen und/oder als Gleichgewichtsprothese zur Sturzprävention und/oder Haltungskontrolle zu verwenden.

Aufgabe der Erfindung war es, ein Mittel bereitzustellen, welches die Nachteile des Standes der Technik nicht aufweist.

Die Aufgabe wird gelöst durch eine mobile Gleichgewichtsprothese, wobei die Gleichgewichtsprothese so ausgebildet ist, dass sie direkt am Körper getragen werden kann und eine Einrichtung, bevorzugt einen Sensor, aufweist, der die Veränderung der Winkelgeschwindigkeit in zwei Ebenen des Raumes von Vorwärts- und/oder Rückwärtsbewegungen des Körpers bevorzugt mittels Gyrometer bestimmt und eine weitere Einrichtung, bevorzugt den Sensor, der die Veränderung der Winkelgeschwindigkeit von Seitwärtsbewegungen des Körpers bevorzugt mittels Gyrometer bestimmt, wobei die Höhe der Signale der Winkelgeschwindigkeitsbestimmung proportional zur Aktivität von Stimulatoren ist, und innerhalb auf den Bewegungsablauf bezogener Grenzen der Werte der Winkelgeschwindigkeit die Aktivierung der Stimulatoren unterbleibt.

Die anmeldungsgemäße Lehre weist gegenüber dem Stand der Technik mehrere Vorteile auf:
- Abkehr vom technisch Üblichen
- neue Aufgabenstellung (gleichzeitiges Lösen des Problems von Gleichgewichtsstörungen und als Gleichgewichtsprothese zur Sturzprävention oder Haltungskontrolle)
- Vorliegen eines seit langem ungelösten, dringenden Bedürfnisses für die Lösung des mit der Erfindung gelösten Problems
- bisheriges vergebliches Bemühen der Fachwelt
- die Einfachheit der Lösung spricht für erfinderische Tätigkeit, insbesondere da sie kompliziertere Lehren ersetzt
- Entwicklung der wissenschaftlichen Technik ging in eine andere Richtung
- entwicklungsstraffende Leistung
- Fehlvorstellungen der Fachwelt über die Lösung des entsprechenden Problems (Vorurteil)
- technischer Fortschritt, wie z. B.: Verbesserung, Leistungssteigerung, Verbilligung, Ersparnis an Zeit, Material, Arbeitsstufen, Kosten oder schwer beschaffbaren Rohstoffen, erhöhte Zuverlässigkeit, Beseitigung von Fehlern, Qualitätshebung, Wartungsfreiheit, größere Effektivität, höhere Ausbeute, Vermehrung der technischen Möglichkeiten, Bereitstellung eines weiteren Mittels, Eröffnung eines zweiten Weges, Eröffnung eines neuen Gebietes, erstmalige Lösung einer Aufgabe, Reservemittel, Alternativen, Möglichkeit der Rationalisierung, Automatisierung oder Miniaturisierung oder Bereichung des Arzneimittelschatzes
- Irrtum in Entgegenhaltungen
- junges Gebiet der Technik
- Kombinationserfindung, d.h. mehrere bekannte Elemente werden zu einer Kombination zusammengeführt, die einen überraschenden Effekt aufweist
- Lizenzvergabe
- Lob der Fachwelt und
- wirtschaftlicher Erfolg.

Überraschenderweise weist die erfindungsgemäße Vorrichtung nicht die Nachteile des Standes der Technik auf. Es wird ein alltagstaugliches mobiles Gerät zur individuellen Unterstützung des Gleichgewichtssystems in allen Alltagssituationen zur Verfügung gestellt. Dieses Gerät ist in der Lage, willkürliche Bewegungen des Trägers von Bewegungen zu unterscheiden, die durch Gleichgewichtsstörungen verursacht werden.

Demgemäß wird durch die erfindungsgemäße Lehre ein mobiles, programmierbares Gerät zur Kompensation fehlender Gleichgewichts- und/oder Orientierungsinformationen in alltäglichen Situationen zur Verfügung gestellt, mit deren Hilfe das Gehirn die Körperhaltung bestimmen und eine Korrektur einleiten kann. Vorteilhafterweise misst die Mess- und Prozesseinheit des Gerätes die Veränderung der Winkelgeschwindigkeit (Corioliskraft) des Körpers oder eines Körperteils in zwei Ebenen des Raumes (x; y) mithilfe von Gyrometern. Es war völlig überraschend, dass Gyrometer verwendet werden können, um den Gleichgewichtssinn eines Menschen zu trainieren.

Am Körper angebrachte Stimulatoren werden in einer Vorzugsvariante der Erfindung aktiviert, wenn sich das Körperteil, an dem das Gerät getragen wird, schneller als durch ein bewegungsspezifisches, persönliches Programm zugelassen, bewegt. Das Programm ist hierbei in einer vorteilhaften Ausführungsform eine Kombination von Signalabschwächern, die den vier Ausgängen der Gyrometer (hinten, vorn, links, rechts) nachgeschaltet sind. Es kann jedoch auch vorteilhaft sein, wenn den vier Ausgängen der Gyrometer Signalverstärker nachgeschaltet werden.

Das Programm ist jeweils spezifisch für eine Tätigkeit oder einen Bewegungsablauf eines individuellen Trägers. Das für die Kontrolle der Körperhaltung geeignete Programm wird für die entsprechende Tätigkeit oder den Bewegungsablauf vom Träger beispielsweise manuell oder mittels Sprachsteuerung oder durch das Gerät selbständig aktiviert, indem beispielsweise durch das Gerät Bewegungsabläufe mithilfe der Auswertung von Hirn- oder Muskelaktivitäten erkannt werden. Durch diese bevorzugte Programmwahl erfolgt eine spezifische Anpassung der Stärke der Abschwächung oder aber auch der Aktivierung oder Erhöhung der Ausgangssignale der Gyrometer, die ein Muster der Ansprechschwellen der Stimulatoren hervorruft, wodurch Aktivitäten und Tätigkeiten im Rahmen der Willkürmotorik stimulationsfrei bleiben.

Weitere vorteilhafte Ausgestaltungsformen der Erfindung ergeben sich aus den Unteransprüchen.

Vorzugsvarianten des Gerätes könnten beispielsweise in der manuellen Bedienung des Programmwahlschalters bestehen oder in der Programmwahl über eine Sprachsteuerung, weiterhin ist die Auswahl des Programms durch die Auswertung von Aktivitäten der Muskulatur oder des Nervensystems möglich.

In einer weiteren Vorzugsvariante der Erfindung wird die Umsetzung von Programmen durch die Kombination von elektrischen Widerständen, die mit dem Programmwahlschalter und den Stimulatoren verbunden sind, realisiert.

Bei einer weiteren Vorzugsvariante ist die Umsetzung von Programmen mithilfe einer Software, die eine Kombination von Abschwächungen in einem Mikroprozessor speichert, vorgesehen.

Weiterhin kann es vorteilhaft sein, wenn die Ausgabe der stimulationen durch Vibrationsstimulatoren erfolgt.

Weiterhin kann es auch bevorzugt sein, dass die Stimulation durch elektrische Reizung auf der Körperoberfläche bzw. von motorischen Nerven bzw. der Muskulatur realisiert wird.

Des weiteren kann es bevorzugt sein, wenn die Stimulation durch Reizung von sensorischen Nerven bzw. von Sinnesorganen oder Teilen dieser erfolgt.

Das Gerät kann bevorzugt am Kopf, Oberkörper, Oberschenkel, Unterschenkel, Oberarm, Unterarm an dem Patienten befestigt werden.

In einer weiteren Vorzugsvariante der Erfindung ist vorgesehen, dass die Befestigung am Körper mit einem Gummiband oder mit einem gummifaserhaltigen Textilband erfolgt. Die Befestigung am Körper kann selbstverständlich auch über ein Ledergeschirr, Textilgeschirr oder über ein Synthetikledergeschirr erfolgen.

Die Erfindung betrifft auch ein Verfahren zum mobilen Gleichgewichtstraining, wobei mittels eines Sensors die Veränderung der Winkelgeschwindigkeit in zwei Ebenen des Raumes von Vorwärt- und/oder Rückwärtsbewegungen des Körpers und/oder die Veränderung der Winkelgeschwindigkeit von Seitwärtsbewegungen des Körpers bestimmt werden, wobei insbesondere Gyrometer einsetzbar sind und wobei das von den Sensoren ermittelte Signal der Winkelgeschwindigkeitsmessung abgeschwächt wird.

Im Folgenden soll die Erfindung anhand eines Beispiels näher erläutert werden, ohne auf dieses Beispiel beschränkt zu sein:

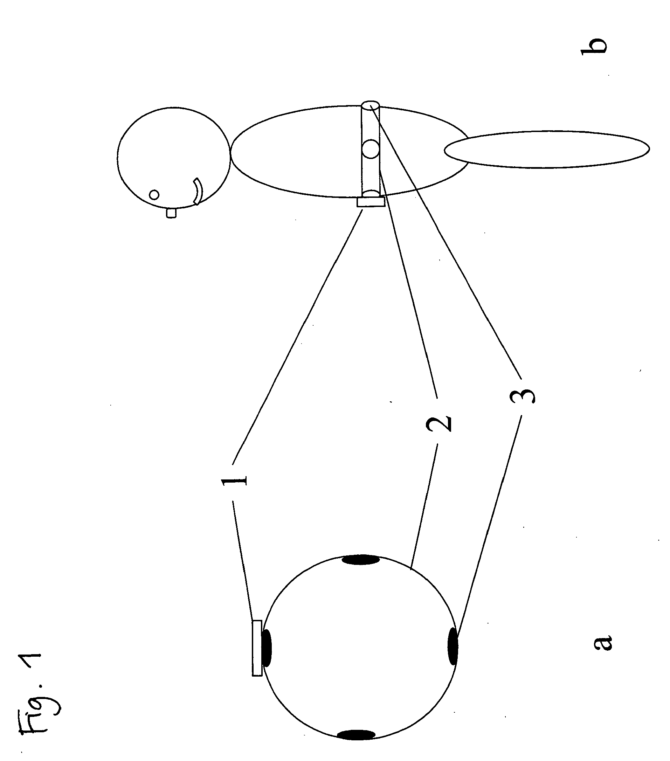

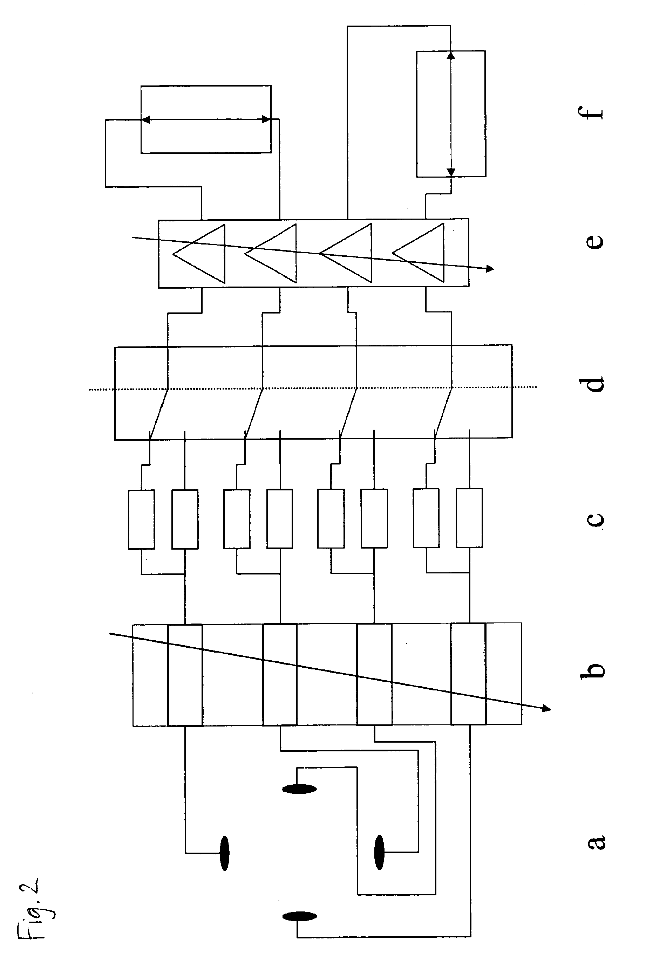

Der in Abb. 1 dargestellte Typ der Vorrichtung stellt eine bevorzugte Variante der Erfindung dar. Dabei werden Vibrationsstimulatoren und eine Mess- und Prozesseinheit an einem Band um die Körpermitte getragen. In Abb. 2 ist das Funktionsschaltbild einer bevorzugten Variante der Erfindung dargestellt. Die Mess- und Prozesseinheit enthält einen Sensor zur Messung der Winkelgeschwindigkeit von Bewegungen in Vorwärts- und Rückwärtsrichtung sowie einen Sensor zur Messung der Winkelgeschwindigkeit von Seitwärtsbewegungen (Abb. 2 Abschnitt f). Das Signal wird kanalspezifisch (Kanal = Bewegungsrichtung) verstärkt (Abb. 2 Abschnitt e). Die Höhe der Verstärkung ist mit einem Potentiometer regelbar, um den Stimulus an die Sensitivität des Trägers anzupassen. An einem Programmwahlschalter (Abb. 2 Abschnitt d) wählt der Träger ein, dem Bewegungsablauf angemessenes Programm aus.

Die Programme beinhalten Informationen über die Höhe der Ansprechschwellen der einzelnen Stimulatoren während bestimmter Aktivitäten oder Tätigkeiten des Trägers. Dieses aktivitäts- oder tätigkeitsspezifische Muster der Schwellen der Stimulatoren entspricht dem Muster, das beim gesunden Menschen während der entsprechenden Verhaltensweise vorliegt. Eine Variante, wie das Muster der Schwellen in einem speziellen Programm umgesetzt wird, ist die Kombination von elektrischen Widerständen, die mit dem Programmwahlschalter und den Stimulatoren verbunden sind (Abb. 2 Abschnitt c).

Die Anpassung an die Leistungsfähigkeit des Trägers hinsichtlich der Aufrechterhaltung des Gleichgewichtes in einer spezifischen Situation wird zusätzlich durch einen Substraktor wahlweise gleichzeitig und gleichmäßig oder separat für die Schwellen aller Stimulatoren erfolgen. Eine Möglichkeit der Umsetzung dieser Anpassung wäre ein stimulatorspezifisches, Potentiometer (Abb. 2 Abschnitt b).

Entsprechend des Funktionsablaufes in Abb. 2 ist die Stärke der Vibration proportional zur Änderung der Winkelgeschwindigkeit und erfolgt an den Stimulatoren, die der Richtung der überschwelligen Bewegung entsprechen.

## Patentansprüche

1. Mobile Gleichgewichtsprothese,
**dadurch gekennzeichnet, dass**
die Gleichgewichtsprothese so ausgebildet ist, dass sie direkt am Körper getragen werden kann und eine Einrichtung, bevorzugt einen Sensor, aufweist, der die Veränderung der Winkelgeschwindigkeit in zwei Ebenen des Raumes von Vorwärts- und/oder Rückwärtsbewegungen des Körpers bevorzugt mittels Gyrometer bestimmt und eine weitere Einrichtung, bevorzugt den Sensor, der die Veränderung der Winkelgeschwindigkeit von Seitwärtsbewegungen des Körpers bevorzugt mittels Gyrometer bestimmt, wobei die Signale der Winkelgeschwindigkeitsbestimmung proportional zur Aktivität von Stimulatoren ist, und innerhalb auf den Bewegungsablauf bezogener Grenzen der Werte der Winkelgeschwindigkeit die Aktivierung der Stimulatoren unterbleibt.

2. Prothese nach dem vorhergehenden Anspruch,
**dadurch gekennzeichnet, dass**
diese am Kopf, Oberkörper, Oberschenkel, Unterschenkel, Oberarm und/oder Unterarm befestigbar ist.

3. Prothese nach einem der vorhergehenden Ansprüche,
**dadurch gekennzeichnet, dass**
sie mithilfe eines Gummibandes, eines gummifaserhaltigen Textilbandes, eines Ledergeschirrs, eines Textilgeschirrs oder eines Synthetikledergeschirrs am Körper positionierbar und/oder befestigbar ist.

4. Prothese nach einem der vorhergehenden Ansprüche,
**dadurch gekennzeichnet, dass**
sie einen manuell bedienbaren Programmschalter aufweist.

5. Prothese nach einem der vorhergehenden Ansprüche,
**dadurch gekennzeichnet, dass**
die Programmwahl mittels Sprachsteuerung und/oder durch Auswertung von Aktivitäten der Muskulatur oder des Nervensystems erfolgt.

6. Prothese nach einem der vorhergehenden Ansprüche,
**dadurch gekennzeichnet, dass**
die Umsetzung von Programmen durch Kombination von elektrischen Widerständen, die mit dem Programmwahlschalter und Stimulatoren verbunden sind, erfolgt.

7. Prothese nach einem der vorhergehenden Ansprüche,
**dadurch gekennzeichnet, dass**
die Umsetzung von Programmen mithilfe von Software erfolgt, die eine Kombination von Abschwächungen der Signale in einem Mikroprozessor speichert.

8. Prothese nach einem der vorhergehenden Ansprüche,
**dadurch gekennzeichnet, dass**
die Stimulatoren Vibrationsstimulatoren sind, Stimulatoren, die durch elektrische Reizung auf der Körperoberfläche angesprochen werden, durch elektrische Reizung von motorischen Nerven bzw. der Muskulatur und/oder durch elektrische Reizung von sensorischen Nerven bzw. Sinnesorganen oder Teilen dieser.

9. Verwendung der Gleichgewichtsprothese nach einem der Ansprüche 1 bis 8 zum Gleichgewichtstraining eines Körpers.

10. Verfahren zum mobilen Gleichgewichtstraining eines Körpers,
**dadurch gekennzeichnet, dass**
mittels eines Sensors die Veränderung der Winkelgeschwindigkeit in zwei Ebenen des Raumes von Vorwärts- und/oder Rückwärtsbewegungen des Körpers und/oder die Veränderung der Winkelgeschwindigkeit von Seitwärtsbewegungen des Körpers bestimmt werden, wobei insbesondere Gyrometer einsetzbar sind und wobei das von den Sensoren ermittelte Signal der Winkelgeschwindigkeitsmessungen abgeschwächt wird.

11. Verwendung eines Gyrometers bei mobilen Gleichgewichtsprothesen.
